# EUROPEAN PATENT SPECIFICATION

(11) **EP 3 721 928 B1**
(45) Date of publication and mention of the grant of the patent: **09.08.2023**
(21) Application number: 19167893.7
(22) Date of filing: 08.04.2019
(51) Int. Cl.: A61M 11/06, A61M 15/00, A61M 16/06

(54) **NEBULIZER FOR AEROSOLIZING A LIQUID WITH COMFORTABLE NOSE PIECE**
ZERSTÄUBER ZUR AEROSOLISIERUNG EINER FLÜSSIGKEIT MIT KOMFORTABLEM MUNDSTÜCK
ATOMISEUR POUR PULVÉRISER UN LIQUIDE AVEC UNE PARTIE AVANT CONFORTABLE

(43) Date of publication of application: 14.10.2020
(73) Proprietor: Air Liquide Medical Systems S.R.L., 20158 Milano (IT)
(72) Inventor: RUOCCO, Alessandra, 25073 Bovezzo (IT); ALBERICI, Luca, 25073 Bovezzo (IT); SANDONI, Giuseppe, 25073 Bovezzo (IT)
(74) Representative: Air Liquide

(56) References cited:
- EP-A1- 2 732 881
- EP-A1- 3 441 097
- CN-U- 203 954 452

## Description

The present disclosure concerns a nebulizer, especially a nebulizer comprising a breath-enhanced valve system, useable in aerosoltherapy comprising a comfortable nasal interface, also called nasal piece or nosepiece, for administrating an aerosol to the nostrils of a patient.

Nebulizing devices or apparatuses, commonly called nebulizers, are devices useable for delivering an inhalation therapy, i.e. an aerosoltherapy, to a patient in need thereof.

Nebulizers are used in combination with a source of gas, typically compressed air, for nebulizing a drug solution thereby obtaining a mist containing droplets having suitable sizes allowing them reaching the lower airways of the patient that has to inhale said mist in the course of his/her aerosoltherapy. An aerosoltherapy is usually provided to the patient by means of a respiratory interface, typically a mouth mask or mouth piece delivering the aerosol only to the mouth of the patient, or an oro-nasal mask covering the nose and the mouth of the patient for delivering the aerosol to both of them.

Examples of such nebulizers are provided by EP-A-3441097, US-A-2011 /253134, DE-A-19902847, GB-A-2358356, and WO-A-03/053500

Patent document EP2732881A1 is hereby acknowledged .

Sometimes, the aerosol has to be delivered only to the nose of the patient, such as in some cough treatments. In that case, a specific nasal interface, also called "nasal piece" or a "nose piece", is needed.

The problem is that few nasal interfaces for nebulizers exist.

Actually, existing nasal interfaces are either not conceived for delivering an aerosol, namely a gas/liquid mixture containing a gas or a gas mixture, and liquid droplets containing one or several compounds, such as drugs, dissolved or in suspension into a liquid, or, when they adapted to nebulizers, they are generally not comfortable.

More precisely, existing nasal interfaces for nebulizers, that are today available on the market, are made of rigid polymeric materials, such as polycarbonate (PC) or polypropylene (PP). However, using rigid polymeric material(s) leads to nasal interfaces that are not comfortable for the patients and further that do not ensure a good gas sealing, i.e. gas tightness, when the nasal interface is positioned on or in the patient's nose, thereby leading to a lack of efficiency of the nebulizer.

Ensuring an efficient seal, i.e. gas tightness, between the nasal interface and the nose/nostrils of the patient is very important in the case of a nebulizer comprising a breath-enhanced valve system including an inhalation valve and an expiration valve. Indeed, in this kind of nebulizer, the negative pressure generated during the inhalation of the patient, opens the inhalation valve, thereby providing an additional flow of air that activates a double Venturi effect that increases the nebulization rate only during inhalation phases. Hence, if the tightness is insufficient, i.e. if leaks exist, the breath-enhanced valve system does not work properly, i.e. cannot be activated, due to undesired air ingresses due to the lack of gas sealing.

A goal of the present technology is to propose a nebulizer, especially a nebulizer comprising a breath-enhanced valve system, comprising an improved nasal interface providing a greater comfort of use for the patient as well as an efficient gas tightness compared to existing nasal interfaces useable in aerosoltherapy, especially those made of rigid polymeric material(s).

A solution according to the present technology is a nebulizer for aerosolizing a liquid, in particular a liquid drug, comprising :
- a nebulizer body comprising :
   - a reservoir part for receiving a liquid to be aerosolized,
   - an aerosol-generating system for generating an aerosol containing said liquid,
   - and a nebulization chamber for receiving aerosol generated by the aerosol-generating system, and
- a nasal interface comprising a hollow body defining an inner chamber, and two nasal prongs to be inserted, in use, into the nostrils of a user, each nasal prong comprising an inner channel in fluid communication with the inner chamber of said hollow body of the nasal interface,
and wherein :
- the nasal interface is detachably fixed to the nebulizer body,
- the inner chamber of the hollow body of the nasal interface is in fluid communication with the nebulization chamber of the nebulizer body for receiving aerosol provided by the nebulization chamber, and
- hollow body and the two nasal prongs of the nasal interface are made of a flexible soft material.

Depending on the embodiment, a nebulizer according to the present technology can comprise one or several of the following additional features:
- the flexible soft material comprises or is silicone.
- the nasal interface is entirely made of said flexible soft material.
- the nasal interface is made in one-piece of said flexible soft material, preferably molded in one-piece.
- the nasal interface is preferably available in different sizes, for instance in 3 sizes such as small, medium and large, to better fit to any nose morphology, e.g. infant, teenagers, adults, women, men...
- the hollow body and the two nasal prongs of the nasal interface are shaped/configured to fit with, i.e. to match, the inner walls of the nostrils of the patient thereby providing a gas sealing in-between.
- the nasal interface comprises a proximal end fixed to the nebulizer body.
- the proximal end of the nasal interface comprises coupling means for attaching/coupling the nasal interface to the nebulizer body.
- the coupling means comprise snap-fit mechanism or the like.
- the nasal prongs each comprise a distal free end comprising an enlarged head to be inserted, in use, into a nostril of the patient.
- the enlarged heads of the nasal prongs have a generally tronconical shape.
- each nasal prong comprises an inner channel terminated by an outlet orifice.
- the enlarged heads of the nasal prongs comprise the outlet orifices.
- each nasal prong comprises an inner channel axially-arranged into each nasal prong.
- each nasal prong comprises a base part integral with the hollow body of the nasal interface.
- each base part of each nasal prong has an elongated shape forming a gas conduct.
- each nasal prong comprises an enlarged head arranged on a base part.
- the nasal prongs project away from the hollow body of the nasal interface.
- it further comprises a breath-enhanced valve system that comprises:
   i) a one-way inspiration valve allowing air entering into the nebulization chamber, and
   ii) a one-way expiration valve allowing gas exiting the nebulization chamber, especially CO₂-containing gas, during the expiration phases of the patient.
- it further comprises a cover forming and/or closing the top of the nebulizer body, especially arranged above the nebulization chamber.
- the cover is detachably fixed/attached to the nebulizer body.
- the cover comprises the breath-enhanced valve system.
- the one-way inspiration valve is configured for allowing gas, such as ambient air, to enter into the nebulization chamber of the nebulization body, during the inspiration phases of the patient, but for preventing any exit/escape of gas through said one-way inspiration valve during the expiration phases of the patient.
- the one-way expiration valve is configured for allowing gas, especially CO₂-containing gas, exiting the nebulization chamber during the expiration phases of the patient, but for preventing any entry of gas through said one-way expiration valve during the inspiration phases of the patient.
- the one-way expiration valve is located between the one-way inspiration valve and the nasal interface, when the cover is integrally fixed to the nebulizer body.
- the reservoir part comprises a liquid tank for receiving a liquid to be aerosolized.
- the liquid tank has a cup-like shape.
- the aerosol-generating system comprises a nozzle element comprising a nozzle orifice and a deflector element facing the nozzle orifice of the nozzle element.
- the aerosol-generating system comprises a nozzle element with an orifice for accelerating the air delivered by a source of compressed air, thereby creating a Venturi or suction effect pulling some liquid solution out of the liquid tank.
- the aerosol-generating system comprises deflector element for atomizing the liquid thereby creating a mist containing liquid droplets.
- the nebulizer body further comprises an axially-arranged inner tube element, i.e. a gas conduct or the like, comprising a lower end projecting into the liquid tank of the reservoir part.
- the lower end of the inner tube element terminates close to the bottom of the liquid tank.
- the reservoir part further comprises an inner conduct projecting axially and upwardly into the liquid tank.
- the axially-arranged inner tube element of the nebulizer body is arranged as a sleeve around a downstream end of the inner conduct of the reservoir part.
- the axially-arranged inner tube element of the nebulizer body is spaced from the downstream end of the inner conduct of the reservoir part by a spacing.
- the nozzle element and the deflector element of the aerosol-generating system are arranged into the lumen of the inner tube element of the nebulizer body.
- the inspiration valve of the cover is facing and in fluid communication with the air inlet located at the upper end of the axially-arranged inner tube element.
- the nebulization chamber is located above the liquid tank.
- the cover is press-fitted, snap-fitted or similarly detachably affixable to the nebulizer body.
- the cover comprises a grip portion.
- the cover comprises a grip portion, such as a textured area, preferably arranged on its outer wall, that allows the user to easily decouple the cover from the nebulizer body.
- the nebulizer body, the reservoir part band/or the cover are made of polymer, preferably a rigid polymer, such as PP, PC, ABS, PA, PSU or any other suitable plastic material.

Further, in relation to the present disclosure
- an "aerosol" is a mist containing droplets of liquid, for instance a liquid drug, and a gas, such as air,
- a "gas" is formed of a unique compound (i.e. a pure gas) or of several compounds (i.e. gas mixture) that is/are in gaseous form.
- a "liquid drug" or a "drug-containing solution" is a solution that comprises one or several liquid compounds and optionally one or several solid compounds dissolved, suspended or similarly incorporated in said one or several liquid compounds.

A preferred embodiment of a nebulizer according to the present disclosure is shown in the enclosed Figures, among which:
- Figure 1 represents a general view of an embodiment of a nebulizer according to the present disclosure
- Figure 2 shows the nebulizer of Figure 1, when used by a patient,
- Figure 3 represents the flows of gases in the nebulizer of Figure 1, during an inspiration phase of the patient,
- Figure 4 represents the flows of gases in the nebulizer of Figure 1, during an expiration phase of the patient, and
- Figure 5 represents a sectional view of the nebulizer of Figure 1.

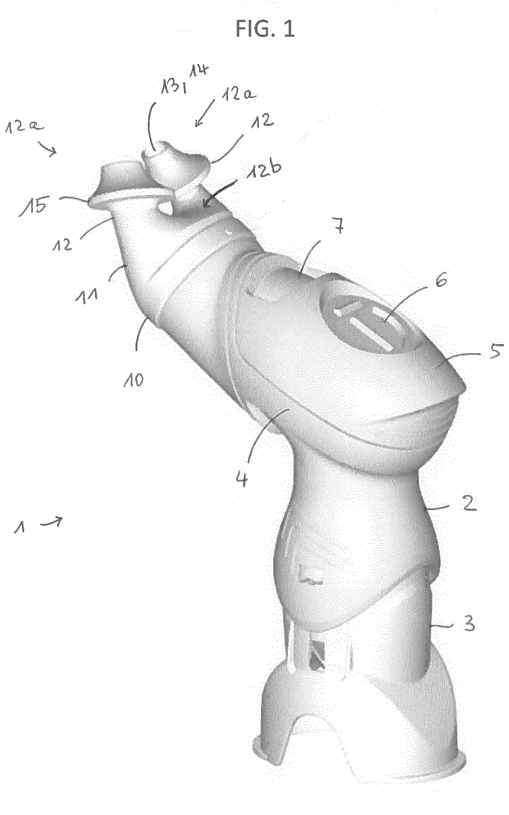

Figure 1 represents a general view of an embodiment of a nebulizer 1 according to the present disclosure designed for aerosolizing a liquid, such as a drug useable in aerosoltherapy.

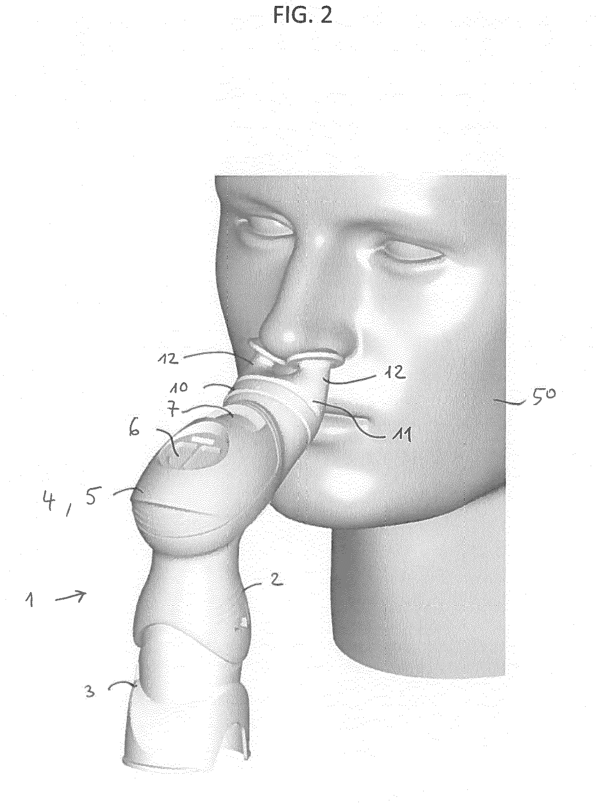

The nebulizer 1 comprises a nebulizer body 2 comprising a reservoir part 3 for receiving a liquid to be aerosolized, an aerosol-generating system (not shown) for generating an aerosol in aerosolizing the liquid provided by the reservoir part 3, a nebulization chamber 4 for receiving the aerosol generated by the aerosol-generating system, and a nasal interface 10 for delivering the aerosol to the nostrils of the patient 50, when the nasal interface 10 is inserted in the nose of the patient, as shown in Figure 2.

Those different parts or elements are detailed below.

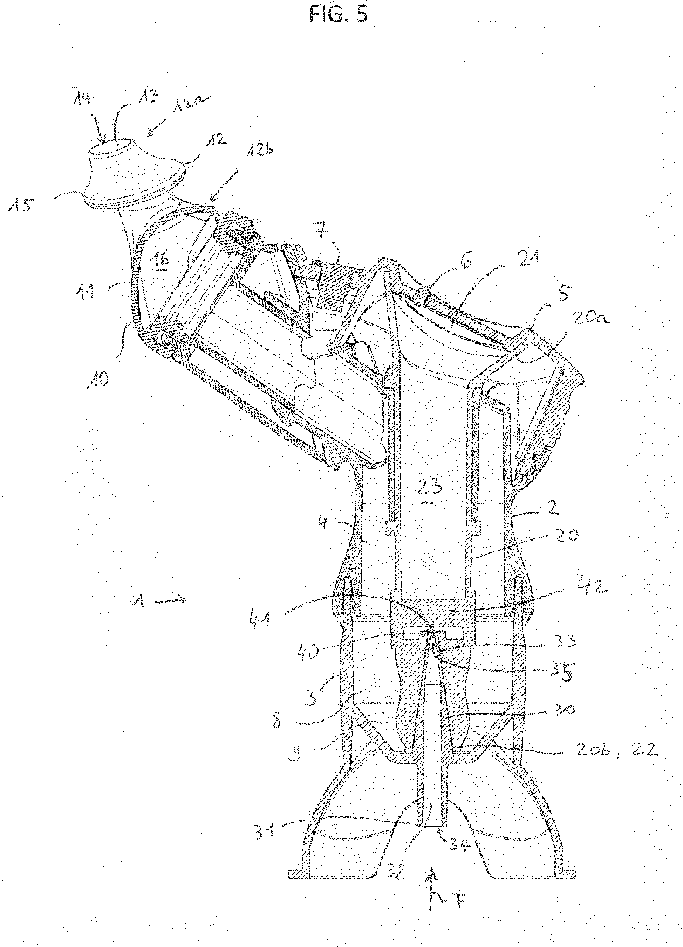

The reservoir part 3 forms a lower part of the nebulizer 1 and is designed for receiving and containing a liquid to be aerosolized, in particular a drug-containing solution. Preferably, the liquid to be aerosolized is stored in a liquid tank 8 arranged in the reservoir part 3 as shown in Figure 5. Preferably, the reservoir part 3 is detachably attached to the nebulizer body 2, for instance they are snap-fitted or threadedly-coupled together, or similarly adapted for repeated attachment/detachment.

As shown in Figure 5, the reservoir part 3 comprises an inner liquid tank 8, also called liquid vessel or compartment, for receiving the liquid 9 to be aerosolized, such as a drug-containing liquid. Preferably, the tank 8 of the reservoir part 3 of the nebulizer 1 has a "cup-like" shape.

The reservoir part 3 further comprises an inner conduct 30 arranged so as to project axially and upwardly into the liquid tank 8 of the reservoir part 3, as shown in Figure 5.

Inner conduct 30 is arranged at the center of the liquid tank 8 and preferably made integral and in one-piece at least with the liquid tank 8 of the reservoir part 3.

The role of inner conduct 30 is to convey a pressurized gas (arrow F) delivered by a gas source (not shown), such as compressed air, fluidly connected to the upstream end 31 of inner conduct 30 that comprises an inlet 34. The compressed gas travels into the lumen or inner passage 32 of inner conduct 30 and is delivered by the downstream end 33 of inner conduct 30 that comprises an outlet orifice 35, for generating the mist or aerosol as explained below.

The nasal interface 10 comprises a hollow body 11 defining an inner chamber 16 for receiving the aerosol and two nasal prongs 12, also called nasal cannulas, to be inserted, in use, into the nostrils of a user 50 as shown in Figure 2.

As shown in Figure 5, each nasal prong 12 comprises an inner channel 13, i.e. a little conduct, in fluid communication with the inner chamber 16 of said hollow body 11 of the nasal interface 10 so that the aerosol contained in said inner chamber can be provided to the inner channel 13 and afterwards delivered to the nostrils of the patient 50 through an outlet orifice 14 located at the free end of each nasal prong 12.

The inner chamber 16 of the hollow body 11 of the nasal interface 10 is further in fluid communication with the nebulization chamber 4 of the nebulizer body 2 for receiving at least a part of the aerosol provided by the nebulization chamber 4 of the nebulizer body 2. In other words, the inner chamber 16 of hollow body 11 is "sandwiched", i.e. arranged, between the nebulization chamber 4 of the nebulizer body 2 and the nasal prongs 12 so that the aerosol can circulates from the nebulization chamber 4 to the nasal prongs 12 via the inner chamber of the nasal interface 10.

The nasal interface 10 useable for delivering a drug-containing mist to a patient 50, is detachably fixed to the nebulizer body 2, for instance by means of a snap-fit mechanism or the like as below explained.

According to the present disclosure, the hollow body 11 and the two nasal prongs 12 of the nasal interface 10 are made of a flexible soft material, preferably silicone or the like. Advantageously, the nasal interface 10 is entirely made of said flexible soft material, preferably molded in one-piece. As the nasal interface 10 of the invention is made of a flexible and soft material, it is more comfortable than existing ones and can better fit to various nose morphologies.

As shown in Fig.1, 3 and 4, the nasal prongs 12 project away from the hollow body 11 of the nasal interface 10. Each of the nasal prongs 12 comprises a distal free end 12a comprising an enlarged head 15 to be inserted, in use, into a nostril of the patient 50, as shown in Fig. 2. The enlarged heads 15 of the nasal prongs 12 have a generally tronconical shape for better matching the shape or profile of the internal walls of the nostrils. Each nasal prong 12 comprises the inner channel 13 or lumen, preferably axially-arranged into each nasal prong 12, and terminated by an outlet orifice 14 arranged in the enlarged head 15 for delivering the aerosol to the nostrils of the patient 50.

Further, each nasal prong 12 comprises a base part 12b integral with the hollow body 11 of the nasal interface 10. Each base part 12b has an elongated shape forming a gas conduct and each enlarged head 15 is arranged on a base part 12b. A good gas tightness is ensured during inhalation so that the inhalation valve 6 of the nebulizer 1 can open and speed up the nebulization rate, when the nebulizer 1 comprises a breath-enhanced valve system 6, 7 as detailed below.

The drug-containing liquid is then micronized at a higher rate only when needed, i.e. during inhalation phases of the patient 50, whereas during expiration phases of the patient 50, the inhalation valve 6 closes and the nebulization rate decreases accordingly.

The nasal interface 10 can be easily fixed and/or detached from the nebulizer body 2 by means of a coupling mechanism, for instance for cleaning operations or when worn out. The nasal interface 10 comprises a proximal end forming the base part 12b, which is connected to the nebulizer body 2, and a distal end that is free, i.e. a distal free end 12a, and comprises the prongs 12.The coupling mechanism can be a snap-fit mechanism, for instance it comprises two grooves on one part that engages two mating snaps on the other part, or any other suitable mechanism.

The nebulization chamber 4 of the nebulizer body 2 is located between the reservoir part 3 and the nasal interface 10 as detailed in Figure 5, and is further in fluid communication with them so as to collect the aerosol generated by the aerosol-generating system 40, 42 and to provide at least a part of said aerosol to the inner chamber 16 of nasal interface 10. In other words, the aerosol generated by the aerosol-generating system 40, 42 travels through the nebulization chamber 4 in the direction of the nasal interface 10.

The nebulization chamber 4 is closed by a detachable top cover 5 including a breath-enhanced valve system 6, 7 comprising two valves, namely an inspiration valve 6 and an expiration valve 7, arranged in parallel, as shown in the embodiment of Figure 5.

In other, the nebulizer body 2 forming the main part of the nebulizer 1 comprises, in the region of the nebulization chamber 4, a top opening that is normally closed by the detachable top cover 5 including the breath-enhanced valve system 6, 7. Said top cover 5 hence constitutes a "lid", "ceiling" or "roof" of the nebulizer body 2, as shown in Figures 1-5. The top cover 5 can be for instance snap-fitted to the body 2.

As detailed in Figure 5, the nebulization chamber 4 is also at least partially arranged around an axially-arranged inner tube element 20 comprising an air inlet 21, at its upper end 20a, an air outlet 22 at its lower end 20b, and an internal passage 23 arranged in-between for conveying ambient air from the air inlet 21 towards the air outlet 22.

Preferably, at least a part of the nebulization chamber 4 is positioned between the peripheral wall of the nebulizer body 2 and the axially-arranged inner tube element 20, and further located above the liquid tank 8.

As shown in Figure 5, when the reservoir part 3 is assembled to the nebulizer body 2, the downstream end 33 of the inner conduct 30 of the reservoir part 3 is inserted into the lumen 23 of the lower end 20b of the axially-arranged inner tube element 20 so that inner tube element 20 forms a sleeve around a part of the inner conduct 30 of the reservoir part 3. The lower end 20b of the axially-arranged inner tube element 20 further projects into the liquid tank 8 of the reservoir part 3 and terminates close to the bottom of the liquid tank 8 containing the liquid 9 to be aerosolized, as shown in Figure 5, preferably at a distance of less than about 2 mm.

The aerosol-generating system 40, 42 arranged in the nebulizer body 2 is used for generating the aerosol, i.e. a mist comprising liquid droplets, and providing it to the nebulization chamber 4. It is arranged in the lumen 23 of the inner tube element 20 of the nebulizer body 2 and comprises, as shown in Figure 5 :
- a nozzle element 40 with a small orifice 41 forming a flow restriction, for instance a restriction orifice 41 having a diameter or dimensions of between about 1 mm to 5 mm. The orifice 41 can have a circular section, an ellipse section or any other suitable shape. Preferably, the nozzle element 40 is axially arranged into lumen 23.
- and a deflector element 42 facing the small orifice 41 of the nozzle element 40, such as a little wall, blade, pin or similar that is arranged into lumen 23, preferably radially-positioned in lumen 23.

For generating the aerosol mist, the liquid 9 contained into tank 8 of the reservoir part 3 should be able to be sucked out of tank 8 and reach the aerosol-generating system 40, 42.

For doing so, it is provided a spacing, on the one hand, between the extremity of the lower end 20b of the axially-arranged inner tube element 20 and the bottom of the liquid tank 8, for instance of less than about 2 mm, and, on the second hand, between the inner wall of the lower end 20b of inner tube element 20 and the outer wall of the downstream end 33 of inner conduct 30 of reservoir part 3, for instance of less than about 1 mm.

The aerosol-generating system 40, 42 can generate the aerosol mist thanks to a Venturi effect. This principle is well-known in the art. In few words, pressurized air is conveyed by the lumen 32 and delivered by the outlet orifice 35 of inner conduct 30, at a high speed and a constant flowrate. This creates a suction effect that moves/sucks the liquid solution 9 out of the tank 8. The liquid solution 9 travels in the spacing, i.e. it is upwardly pulled by the suction effect, and is then delivered by the small orifice 41 of the nozzle element 40 toward the deflector element 42. When the liquid enters into contact with the deflector element 4, a mist of liquid droplets is created by the impact of the liquid on the deflector element 4. The smaller droplets create the desired aerosol mist that exist the lumen 23 of the inner tube element 20, via opening(s) arranged in its tubular wall, and is recovered into the nebulization chamber 4, whereas the larger and/or heavier droplets return by gravity to the liquid tank 8.

The aerosol mist can thereafter be inhaled by the patient via the nasal interface 10.

Generally speaking, all the components of the nebulizer 1, i.e. cover 5, nebulizer body 2..., are preferably made of plastic material(s), such as PP, PC, ABS, PA, PSU or similar materials.

Grip portions can be arranged in the peripheral wall of the nebulizer body 2 for improving the hand grip of the nebulizer 1 by the patient and on the cover 5 for allowing an easy coupling/uncoupling with the nebulizer body.

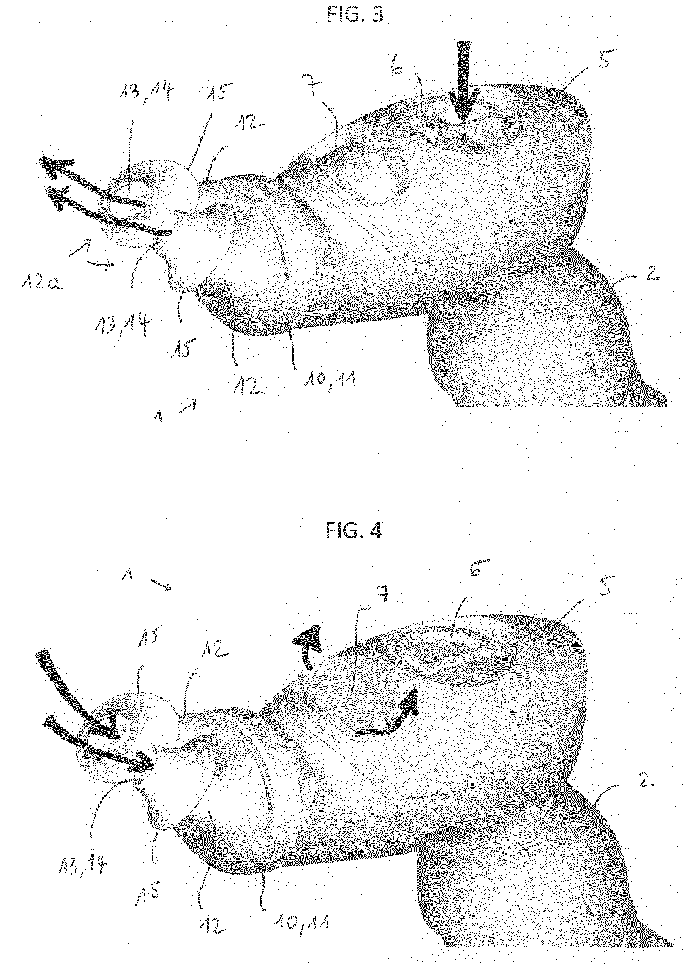

As already mentioned, the cover 5 comprises a breath-enhanced valve system 6, 7 comprising a pair of valves comprising :
- a one-way inspiration valve 6 allowing ambient air entering into the nebulizer 1, during the inspiration phases of the patient 50, as shown in Figure 3, and
- a one-way expiration valve 7 allowing gas exiting the nebulizer 1, during the expiration phases of the patient, especially expired gases that contains CO₂, as shown in Figure 4.

Preferably, the one-way expiration valve 7 is located between the one-way inspiration valve 6 and the nasal interface 10, when the cover 5 is integrally fixed to the nebulizer body 2.

The inspiration valve 6 is arranged on the cover 7 so as to face and be in fluid communication with the air inlet 21 located at the upper end 21a of the axially-arranged inner tube element 20, and opens only during inspiration phases of the patient 50 by action of the negative pressure generated by the patient's inspiration, i.e. during inspiration phases.

The inspiration valve 6 provides an additional flow of ambient air that is used for boosting the nebulization, due to a "double" Venturi effect, and for significantly increasing the amount of aerosol mist formed into the nebulizer and delivered to the nebulization chamber 4, thereby increasing the nebulization rate of the liquid drug 9, i.e. the solution containing the medication. The inspiration valve 6 is a one-way valve allowing air to only travel from the outside to the inside of the nebulizer 1, i.e. entering into the axially-arranged inner tube element 20.

Further, the expiration valve 7 is located next to the inspiration valve 6 on the detachable cover 5, but closer to the nasal interface 10 than the inspiration valve 6.

Said expiration valve 9 is also a one-way valve, that opens by action of the CO₂-containing gases exhaled by the patient 50 that create an overpressure (i.e. pressure > 1 bar) in the nasal interface 10 and at the inlet of the nebulization chamber 4, while the patient is exhaling gases. Said overpressure allows gases to be vented from the nebulizer 1, through the expiration valve 7, when the patient exhales. The on-way expiration valve 7 allows the CO₂-containing gas(es) to travel only from the inside to the outside of the nebulizer 1, i.e. to be vented to the atmosphere.

Thanks to the nebulizer 1 according to the present disclosure, the patient can execute his/her aerosoltherapy by both inhaling and exhaling gases through the nasal interface 10 and the breath-enhanced valve system 6, 7 arranged on the cover 5, thereby limiting the amount of aerosolized drug wasted in the environment as the exit of expired gases is controlled by the one-way expiration valve 7.

The nebulizer 1 of the present disclosure is usable in combination with a source of gas, such as compressed air, for nebulizing a drug-containing solution and thereby obtaining a mist containing droplets of medication having suitable sizes so as to be able to reach the lower airways of the patient that has to inhale said mist in the frame of his/her aerosoltherapy.

## Claims

1. Nebulizer (1) for aerosolizing a liquid comprising :
- a nebulizer body (2) comprising :
. a reservoir part (3) for receiving a liquid to be aerosolized,
. an aerosol-generating system (40,42) for generating an aerosol containing said liquid, and
. a nebulization chamber (4) for receiving aerosol generated by the aerosol-generating system (40, 42),
- a breath-enhanced valve system (6, 7) comprising several valves, and
- a nasal interface (10) comprising a hollow body (11) defining an inner chamber (16), and two nasal prongs (12) to be inserted into the nostrils of a user (20), each nasal prong (12) comprising an inner channel (13) in fluid communication with the inner chamber (16) of said hollow body (11) of the nasal interface (10),
and wherein :
- the nasal interface (10) is detachably fixed to the nebulizer body (2),
- the inner chamber (16) of the hollow body (11) of the nasal interface (10) is in fluid communication with the nebulization chamber (4) of the nebulizer body (2) for receiving aerosol provided by the nebulization chamber (4), and
- the hollow body (11) and the two nasal prongs (12) of the nasal interface (10) are made of a flexible soft material.

2. Nebulizer according to the preceding Claim, **characterized in that** the nasal interface (10) is entirely made of said flexible soft material.

3. Nebulizer according to any one of the preceding Claims, **characterized in that** it the flexible soft material comprises or is silicone.

4. Nebulizer according to any one of the preceding Claims, **characterized in that** each nasal prong (12) comprises an inner channel (13) terminated by an outlet orifice (14).

5. Nebulizer according to any one of the preceding Claims, **characterized in that** the nasal prongs (12) each comprise a distal free end (12a) comprising an enlarged head (15) to be inserted, in use, into a nostril of the patient.

6. Nebulizer according to any one of the preceding Claims, **characterized in that** the enlarged heads (15) of the nasal prongs (12) have a generally tronconical shape.

7. Nebulizer according to any one of the preceding Claims, **characterized in that** the enlarged heads (15) of the nasal prongs (12) comprise the outlet orifices (14).

8. Nebulizer according to any one of the preceding Claims, **characterized in that** the nasal prongs (12) project away from the hollow body (11) of the nasal interface (10).

9. Nebulizer according to any one of the preceding Claims, **characterized in that** the nasal prongs (12) each comprise a base part (12b) integral with the hollow body (11) of the nasal interface (10), each base part (12b) having an elongated shape.

10. Nebulizer according to any one of the preceding Claims, **characterized in that** the enlarged head (15) are arranged on the base parts (12b) of the nasal prongs (12).

11. Nebulizer according to any one of the preceding Claims, **characterized in that** the enlarged heads (15) are configured for matching the inner walls of the nostrils of a user (50).

12. Nebulizer according to any one of the preceding Claims, **characterized in that** it further comprises a breath-enhanced valve system (6, 7) comprising:
i) a one-way inspiration valve (6) allowing air entering into the nebulization chamber (4), and
ii) a one-way expiration valve (7) allowing gas exiting the nebulization chamber (4).

13. Nebulizer according to any one of the preceding Claims, **characterized in that** it further comprises a detachable cover (5) attached to the nebulizer body (2), the one-way inspiration valve (6) and the one-way expiration valve (7) of the breath-enhanced valve system (6, 7) being arranged on said detachable cover (5).

14. Nebulizer according to any one of the preceding Claims, **characterized in that** the aerosol-generating system comprises a nozzle element comprising a nozzle orifice and a deflector element facing the nozzle orifice of the nozzle element.

## Patentansprüche

1. Vernebler (1) zur Vernebelung einer Flüssigkeit, umfassend:
- einen Verneblerkörper (2), der Folgendes umfasst:
• einen Reservoirteil (3) zur Aufnahme einer zu vernebelnden Flüssigkeit,
• ein Aerosolerzeugungssystem (40, 42) zum Erzeugen eines die Flüssigkeit enthaltenden Aerosols und
• eine Vernebelungskammer (4) zur Aufnahme von durch das Aerosolerzeugungssystem (40, 42) erzeugtem Aerosol,
- ein durch Atmung verstärktes Ventilsystem (6, 7), das mehrere Ventile umfasst, und
- eine Nasenschnittstelle (10), die einen hohlen Körper (11), der eine innere Kammer (16) definiert, und zwei Nasen-Prongs (12) umfasst, die in die Nasenlöcher eines Benutzers (20) einzuführen sind, wobei jeder Nasen-Prong (12) einen inneren Kanal (13) in Fluidverbindung mit der inneren Kammer (16) des hohlen Körpers (11) der Nasenschnittstelle (10) umfasst,
und wobei:
- die Nasenschnittstelle (10) lösbar an dem Verneblerkörper (2) befestigt ist,
- die innere Kammer (16) des hohlen Körpers (11) der Nasenschnittstelle (10) zur Aufnahme von Aerosol, das von der Vernebelungskammer (4) bereitgestellt wird, in Fluidverbindung mit der Vernebelungskammer (4) des Verneblerkörpers (2) ist, und
- der hohle Körper (11) und die beiden Nasen-Prongs (12) der Nasenschnittstelle (10) aus einem flexiblen weichen Material hergestellt sind.

2. Vernebler nach dem vorhergehenden Anspruch, **dadurch gekennzeichnet, dass** die Nasenschnittstelle (10) gänzlich aus dem flexiblen weichen Material hergestellt ist.

3. Vernebler nach einem der vorhergehenden Ansprüche, **dadurch gekennzeichnet, dass** das flexible weiche Material Silikon umfasst oder ist.

4. Vernebler nach einem der vorhergehenden Ansprüche, **dadurch gekennzeichnet, dass** jeder Nasen-Prong (12) einen inneren Kanal (13) umfasst, an dessen Ende eine Auslassöffnung (14) angeordnet ist.

5. Vernebler nach einem der vorhergehenden Ansprüche, **dadurch gekennzeichnet, dass** die Nasen-Prongs (12) jeweils ein distales freies Ende (12a) umfassen, das einen vergrößerten Kopf (15) umfasst, der im Gebrauch in ein Nasenloch des Patienten einzuführen ist.

6. Vernebler nach einem der vorhergehenden Ansprüche, **dadurch gekennzeichnet, dass** die vergrößerten Köpfe (15) der Nasen-Prongs (12) eine allgemein kegelstumpfförmige Gestalt haben.

7. Vernebler nach einem der vorhergehenden Ansprüche, **dadurch gekennzeichnet, dass** die vergrößerten Köpfe (15) der Nasen-Prongs (12) die Auslassöffnungen (14) umfassen.

8. Vernebler nach einem der vorhergehenden Ansprüche, **dadurch gekennzeichnet, dass** die Nasen-Prongs (12) von dem hohlen Körper (11) der Nasenschnittstelle (10) weg ragen.

9. Vernebler nach einem der vorhergehenden Ansprüche, **dadurch gekennzeichnet, dass** die Nasen-Prongs (12) jeweils einen Basisteil (12b) umfassen, der mit dem hohlen Körper (11) der Nasenschnittstelle (10) integral ist, wobei jeder Basisteil (12b) eine längliche Form hat.

10. Vernebler nach einem der vorhergehenden Ansprüche, **dadurch gekennzeichnet, dass** die vergrößerten Köpfe (15) an den Basisteilen (12b) der Nasen-Prongs (12) angeordnet sind.

11. Vernebler nach einem der vorhergehenden Ansprüche, **dadurch gekennzeichnet, dass** die vergrößerten Köpfe (15) dazu ausgestaltet sind, sich an die Innenwände der Nasenlöcher eines Benutzers (50) anzupassen.

12. Vernebler nach einem der vorhergehenden Ansprüche, **dadurch gekennzeichnet, dass** er ferner ein durch Atmung verstärktes Ventilsystems (6, 7) umfasst, das Folgendes umfasst:
i) ein Einweg-Inspirationsventil (6), dank dessen Luft in die Vernebelungskammer (4) eintreten kann, und
ii) ein Einweg-Exspirationsventil (7), dank dessen Gas aus der Vernebelungskammer (4) austreten kann.

13. Vernebler nach einem der vorhergehenden Ansprüche, **dadurch gekennzeichnet, dass** er ferner eine abnehmbare Abdeckung (5) umfasst, die an dem Verneblerkörper (2) angebracht ist, wobei das Einweg-Inspirationsventil (6) und das Einweg-Exspirationsventil (7) des durch Atmung verstärkten Ventilsystems (6, 7) an der abnehmbaren Abdeckung (5) angeordnet sind.

14. Vernebler nach einem der vorhergehenden Ansprüche, **dadurch gekennzeichnet, dass** das Aerosolerzeugungssystem ein Düsenelement umfasst, das eine Düsenöffnung und ein der Düsenöffnung des Düsenelements zugewandtes Ablenkungselement umfasst.

## Revendications

1. Nébuliseur (1) destiné à aérosoliser un liquide comprenant :
- un corps de nébuliseur (2) comprenant :
. une partie réservoir (3) destinée à recevoir un liquide devant être aérosolisé,
. un système de génération d'aérosol (40, 42) destiné à générer un aérosol contenant ledit liquide ; et
. une chambre de nébulisation (4) destinée à recevoir l'aérosol généré par le système de génération d'aérosol (40, 42),
- un système de valves pour respiration améliorée (6, 7) comprenant plusieurs valves, et
- une interface nasale (10) comprenant un corps creux (11) définissant une chambre interne (16), et deux canules nasales (12) devant être insérées dans les narines d'un utilisateur (20), chaque canule nasale (12) comprenant un canal interne (13) en communication fluidique avec la chambre interne (16) dudit corps creux (11) de l'interface nasale (10),
et dans lequel :
- l'interface nasale (10) est fixée de façon détachable au corps de nébuliseur (2),
- la chambre interne (16) du corps creux (11) de l'interface nasale (10) est en communication fluidique avec la chambre de nébulisation (4) du corps de nébuliseur (2) pour recevoir un aérosol fourni par la chambre de nébulisation (4), et
- le corps creux (11) et les deux canules nasales (12) de l'interface nasale (10) sont constitués d'un matériau mou souple.

2. Nébuliseur selon la revendication précédente, **caractérisé en ce que** l'interface nasale (10) est entièrement constituée dudit matériau mou souple.

3. Nébuliseur selon l'une quelconque des revendications précédentes, **caractérisé en ce que** le matériau mou souple comprend ou est du silicone.

4. Nébuliseur selon l'une quelconque des revendications précédentes, **caractérisé en ce que** chaque canule nasale (12) comprend un canal interne (13) terminé par un orifice de sortie (14).

5. Nébuliseur selon l'une quelconque des revendications précédentes, **caractérisé en ce que** les canules nasales (12) comprennent chacune une extrémité distale libre (12a) comprenant une tête élargie (15) devant être insérée, à l'usage, dans une narine du patient.

6. Nébuliseur selon l'une quelconque des revendications précédentes, **caractérisé en ce que** les têtes élargies (15) des canules nasales (12) ont une forme généralement tronconique.

7. Nébuliseur selon l'une quelconque des revendications précédentes, **caractérisé en ce que** les têtes élargies (15) des canules nasales (12) comprennent les orifices de sortie (14).

8. Nébuliseur selon l'une quelconque des revendications précédentes, **caractérisé en ce que** les canules nasales (12) se projettent loin du corps creux (11) de l'interface nasale (10).

9. Nébuliseur selon l'une quelconque des revendications précédentes, **caractérisé en ce que** les canules nasales (12) comprennent chacune une partie de base (12b) faisant partie intégrante du corps creux (11) de l'interface nasale (10), chaque partie de base (12b) ayant une forme allongée.

10. Nébuliseur selon l'une quelconque des revendications précédentes, **caractérisé en ce que** les têtes élargies (15) sont disposées sur les parties de base (12b) des canules nasales (12).

11. Nébuliseur selon l'une quelconque des revendications précédentes, **caractérisé en ce que** les têtes élargies (15) sont configurées pour s'adapter aux parois internes des narines d'un utilisateur (50).

12. Nébuliseur selon l'une quelconque des revendications précédentes, **caractérisé en ce qu'**il comprend en outre un système de valves à respiration améliorée (6, 7) comprenant :
i) une valve d'inspiration unidirectionnelle (6) permettant à de l'air d'entrer dans la chambre de nébulisation (4), et
ii) une valve d'expiration unidirectionnelle (7) permettant à du gaz de sortir de la chambre de nébulisation (4).

13. Nébuliseur selon l'une quelconque des revendications précédentes, **caractérisé en ce qu'**il comprend en outre un couvercle détachable (5) attaché au corps de nébuliseur (2), la valve d'inspiration unidirectionnelle (6) et la valve d'expiration unidirectionnelle (7) du système de valves à respiration améliorée (6, 7) étant disposées sur ledit couvercle détachable (5).

14. Nébuliseur selon l'une quelconque des revendications précédentes, **caractérisé en ce que** le système de génération d'aérosol comprend un élément faisant buse comprenant un orifice de buse et un élément faisant déflecteur faisant face à l'orifice de buse de l'élément faisant buse.
